# EUROPEAN PATENT SPECIFICATION

(11) **EP 4 499 602 B1**
(45) Date of publication and mention of the grant of the patent: **29.04.2026**
(21) Application number: 23716933.9
(22) Date of filing: 27.03.2023
(51) Int. Cl.: C07C 227/18, C07C 229/32

(54) **PROCESS FOR OBTAINING DISODIUM TYROSINE BY SPRAY DRYING**
VERFAHREN ZUR GEWINNUNG VON DINATRIUMTYROSIN DURCH SPRÜHTROCKNUNG
PROCÉDÉ PERMETTANT D'OBTENIR DE LA TYROSINE DISODIQUE PAR SÉCHAGE PAR PULVÉRISATION

(30) Priority: 28.03.2022 IT 202200006014
(43) Date of publication of application: 05.02.2025
(73) Proprietor: Flamma SpA, 24040 Chignolo d'Isola (BG) (IT)
(72) Inventor: VERZINI, Massimo, 24040 Chignolo d'Isola (BG) (IT); TRUCCHI, Beatrice, 24040 Chignolo d'Isola (BG) (IT)
(74) Representative: Dragotti & Associati S.P.A.
(86) International application number: PCT/IB2023/052998
(87) International publication number: WO 2023/187602

(56) References cited:
- US-A- 4 734 401
- US-A- 4 956 489

## Description

### TECHNICAL FIELD

The present invention relates to the process of obtaining disodium tyrosine, preferably dihydrate, in a crystalline form, by spray drying technology.

### BACKGROUND OF THE INVENTION

Tyrosine disodium dihydrate is used as a component of food supplements for human use and as an additive for cell culture media.

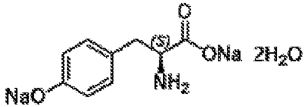

Its remarkable solubility in water makes it difficult to obtain it by common synthesis methods which provide for the dissolution of L-tyrosine in a sodium hydroxide solution and its precipitation through the addition of anti-solvents.

Preliminary tests carried out by the authors made it necessary the addition of 12 kg of ethanol per kg of L-tyrosine, to obtain product precipitation from an aqueous solution of L-tyrosine in sodium hydroxide (39.8% kg/kg of solution). The main disadvantages of this process are represented by the use of a considerable amount of solvent and unsatisfactory yields (66.6%). The procedure is therefore not very sustainable from both an environmental and a production point of view.

Therefore, the present inventors have developed a process which, through spray-drying technology, allows to increase the yield, significantly reduce the process volumes, increase productivity, and avoid the use of organic solvents, with a consequent reduction in costs and lower environmental impact.

US patent US4734401 describes a spray drying process applied to the synthesis of amino acids in general, and specifically to the amorphous form of tyrosine ammonium salt. The technical problem to be overcome with said patent was the need to obtain an amorphous product through a synthesis method that would allow for an increase in process yields and a reduction in the use of solvents compared to conventional methods. The process described in US4734401 patent provides for the preparation of a solution of L-tyrosine (40.0 g/L of solution) in 14% aqueous ammonia (pH 12-13) at 70°C. The solution is subjected to a spray drying process in an air or a nitrogen stream, using an inlet temperature of 210-221°C and an outlet temperature of 106-132°C (preferably 100-125°C), maintained with a solution feed rate of 100 g/minute. US4956489 discloses derivatives of L-tyrosine with improved solubility. Disodium tyrosine is also disclosed. A method for its preparation is however not described.

The object of the present invention, instead, differs from what is described in the state of the art, in that:
- it allows to obtain not an amorphous but a crystalline product,
- the product is obtained as a disodium salt, preferably in dihydrate form, and not as an ammonium salt,
- the inlet and outlet temperatures are preferably maintained between 130-180°C and 80-100°C, respectively, in the process carried out in a nitrogen stream, and between 165-180°C and 80-100°C in the process carried out in an air stream (preferably 165°C and 95°C, respectively).

### SUMMARY OF THE INVENTION

The object of the present invention is to provide a method for producing disodium tyrosine, preferably dihydrate, by a spray drying process. According to a first aspect, the present invention relates to a process for producing disodium tyrosine, preferably dihydrate, comprising the steps of:
a) preparing an aqueous solution of L-tyrosine in sodium hydroxide;
b) subjecting the obtained solution to spray drying in an inert gas atmosphere, preferably nitrogen, or in an air atmosphere.

In a preferred embodiment, the L-tyrosine aqueous solution of step a) is prepared in sodium hydroxide at 28.5-32.5% by weight, preferably at 30% by weight, more preferably at room temperature, according to the Synthesis Scheme reported below.

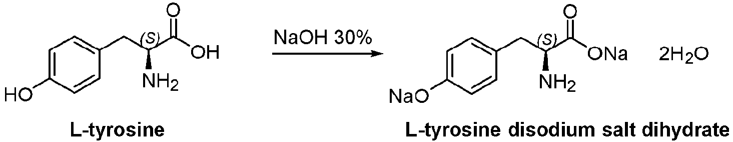

Preferably, water is added to the sodium hydroxide solution to ensure complete dissolution of disodium tyrosine before the spray drying step.

In a further preferred embodiment, the spray drying step b) is carried out in an inert gas stream, preferably in a nitrogen stream.

In said embodiment, the inlet temperature is between 130 and 180°C, and the outlet temperature is between 80 and 100°C.

In a further alternative embodiment, the spray drying step b) is carried out in an air stream.

In said embodiment, the inlet temperature is between 165 and 180°C, preferably 165°C, and the outlet temperature is between 80 and 100°C, preferably 95°C.

Compared to the classic methods for obtaining the product, the process according to the invention allows to reduce the reaction volumes, increase productivity (since the yield is quantitative with minimal mechanical losses), significantly reduce the amount of solvents to be disposed of with undoubted advantages in terms of environmental impact and costs. Furthermore, the method according to the invention preserves the stability of the product, which is prone to oxidation.

### DESCRIPTION OF THE FIGURES

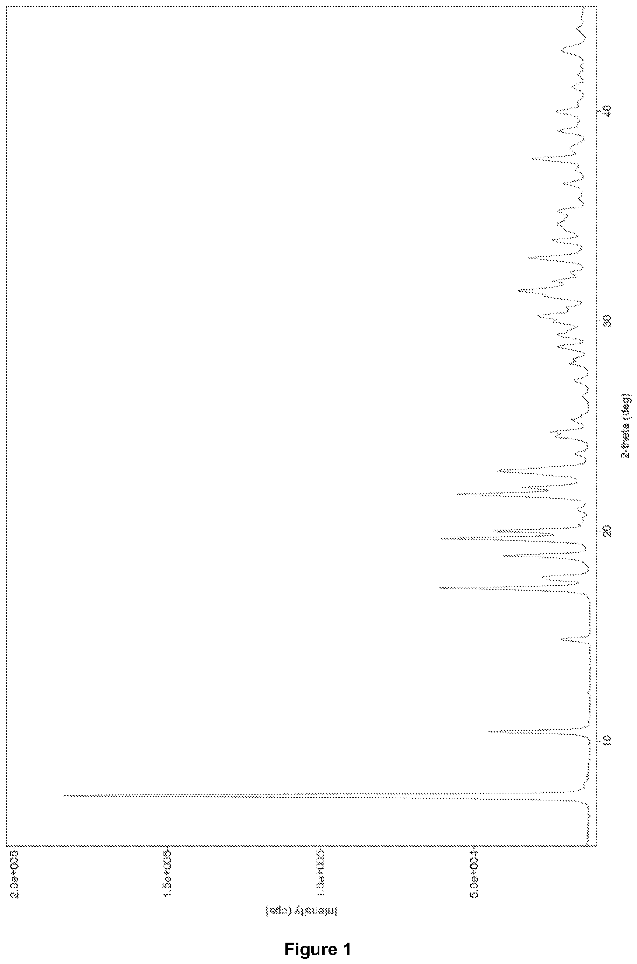

**Figure 1** shows the XRPD (X-Ray Powder Diffraction) spectrum of disodium tyrosine dihydrate obtained in Example 1 (Invention);

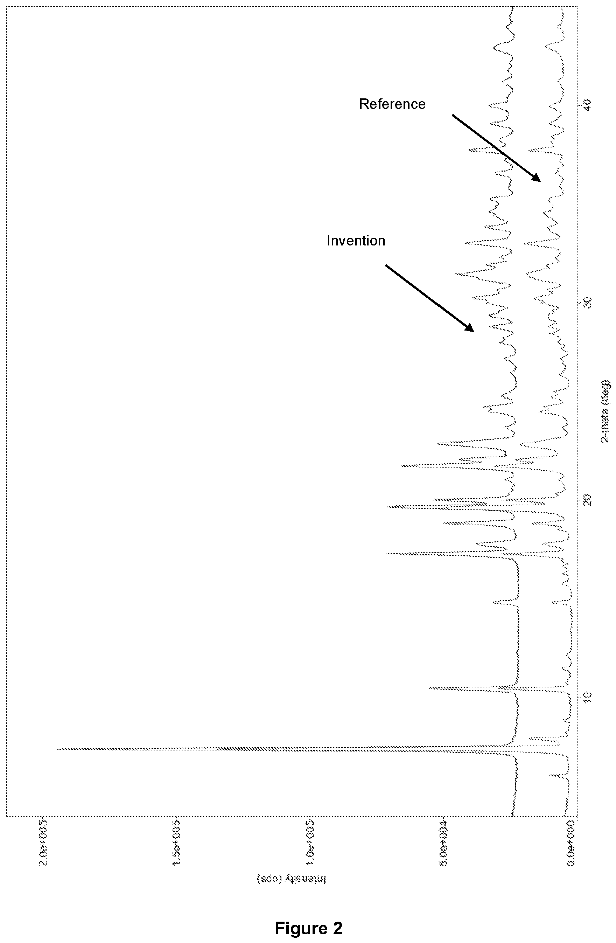

**Figure 2** shows the XRPD spectrum of tyrosine disodium dihydrate from Figure 1 in comparison with the XRPD spectrum of crystalline disodium tyrosine dihydrate obtained by precipitation through addition of a state-of-the-art anti-solvent (Reference).

### DEFINITIONS

Unless otherwise defined, all terms of the art, notations and other scientific terminology used herein are intended to have the meanings commonly understood by those skilled in the art to which this disclosure pertains. In some cases, terms with commonly understood meanings are defined herein for clarity and/or for ready reference; thus, the inclusion of such definitions in the present disclosure should not be construed to represent a substantial difference over what is generally understood in the art.

The terms "**approximately**" and "**about**" used in the text refer to the range of the experimental error that is inherent in the execution of an experimental measurement.

The terms "**comprising**", "**having**", "**including**" and "**containing**" are to be intended as open-ended terms (i.e., meaning "comprising, but not limited to"), and are to be considered as a support also for terms such as "consist essentially of", "consisting essentially of", "consist of", or "consisting of".

The terms "**consist essentially of**", "**consisting essentially of**" are to be intended as semi-closed terms, meaning that no other ingredients affecting the novel features of the invention are included (optional excipients may therefore be included).

The terms "**consists of**", "**consisting of**" are to be intended as closed terms.

The term "**room temperature**" refers to a temperature between 15°C and 25°C, preferably between 20°C and 25°C.

### DETAILED DESCRIPTION OF THE INVENTION

Object of the present invention is a process for producing disodium tyrosine, preferably dihydrate, comprising the steps of:
a) preparing an aqueous solution of L-tyrosine in sodium hydroxide;
b) subjecting the obtained solution to spray drying in an inert gas atmosphere, preferably nitrogen, or in an air atmosphere.

The process according to the invention first provides for the preparation of a basic aqueous solution of L-tyrosine, preferably obtained by adding to L-tyrosine, at room temperature and under stirring, a sodium hydroxide solution at 28.5-32.5% by weight, more preferably at 30% by weight (for example 1.51 kg of 30% NaOH per kg of L-tyrosine), even more preferably followed by the addition of water (for example 1 kg of water per kg of L-tyrosine). The solution is then subjected to a spray drying process, carried out both in inert conditions and in an air stream.

In a preferred embodiment, as regards the spray drying process carried out in a nitrogen stream, inlet temperatures between 130°C and 180°C, and outlet temperatures preferably between 80 and 100°C, were preferably used. The temperature difference reached (comprised between 40 and 80°C) was modulated by varying some parameters, among which the solution feeding speed (varied between 26 and 56 Kg/h) and the type of nozzle used (pressure nozzle or 2-fluid nozzle).

As regards the spray drying process carried out in an air stream, the inlet temperatures are preferably between 165 and 180°C, the outlet ones are preferably between 80 and 100°C. The apparatus which can be used for the purposes of the present invention consists of a turbine atomizer, whose rotation speed is between 16,000 and 18,000 rpm.

The product obtained was analyzed in terms of:
- quality (potentiometric titer determination; optical rotation power; ALPHA color)
- solid state by X-rays;
- particle size (by sieve or laser diffraction analyzer).

The product, tyrosine disodium salt dihydrate, which is preferably obtained with the method according to the invention is:
1) of the desired quality, determined by potentiometric titer (98.0-102.0% w/w), color (APHA color, 1% aqueous solution, 200 Max), optical rotation power (20°C, c = 4 g/100 mL, aqueous solution, as free acid -14.4°/12.6°);
2) with a water content between 12.0 and 16.0% w/w, as determined by Karl Fisher (K.F.);
3) not an amorphous, but a crystalline solid, with a 100 mesh particle size distribution ≥ 95%.

### Experimental Part

### Example 1. Synthesis of disodium tyrosine dihydrate by spray drying process in an air stream according to the invention

37.75 kg of a 30 wt% sodium hydroxide solution (283.13 mol, 2.05 equiv.) was added with stirring at room temperature to 25 kg of L-tyrosine (137.98 mol). 25 kg of water were added to the suspension, and it was kept under stirring for two hours at room temperature until complete dissolution.

The resulting solution was subjected to a spray drying process in an air stream, using an inlet temperature of 165°C and an outlet temperature of 95°C, with a turbine speed of 16,000 rpm. The product obtained at the discharge was cooled to a temperature between 4 and 8°C, then subjected to sieving. The process yield was quantitative (36.0 kg of disodium L-tyrosine dihydrate, 137.98 mol).

The product, analyzed to evaluate its quality, solid state, and particle size, satisfies the expected requirements as evidenced by the results shown below.
Water content (Karl Fisher): 14.2% w/w
APHA color (visual test): <200
Potentiometric titer: 101.4% w/w
Optical rotation power [α]^{D}₂₀ (20°C, c = 4 g/100 mL, free acid, aqueous solution): -14.2°
Particle size, by 100 mesh sieving, 99.3%
Solid state determined by X-rays: crystalline solid.

## Claims

1. A process for producing disodium tyrosine, preferably dihydrate, comprising the steps of:
a) preparing an aqueous solution of L-tyrosine in sodium hydroxide;
b) subjecting the obtained solution to spray drying in an inert gas atmosphere, preferably nitrogen, or in an air atmosphere.

2. The process according to claim 1, **characterized in that** step a) is carried out at room temperature.

3. The process according to claim 1 or 2, **characterized in that** the sodium hydroxide solution is at 28.5%-32.5% by weight, preferably at 30% by weight.

4. The process according to any one of the preceding claims, **characterized in that** an amount of water is added to the sodium hydroxide solution, preferably an amount of water equal to the initial amount of L-tyrosine.

5. The process according to any one of the preceding claims, **characterized in that** step b) is carried out in an atmosphere of inert gas, preferably nitrogen.

6. The process according to claim 5, **characterized in that** the inlet temperature is between 130°C and 180°C and the outlet temperature is between 80 and 100°C.

7. The process according to any one of claims 1 to 4, **characterized in that** step b) is carried out in an air atmosphere.

8. The process according to claim 7, **characterized in that** the inlet temperature is between 165°C and 180°C, preferably 165°C, and the outlet temperature is between 80 and 100°C, preferably 95°C.

## Patentansprüche

1. Prozess zum Herstellen von Dinatriumsalzen von Tyrosin, vorzugsweise Dihydrat, umfassend die folgenden Schritte:
a) Zubereiten einer wässrigen Lösung von L-Tyrosin in Natriumhydroxid;
b) Unterziehen der erhaltenen Lösung einer Sprühtrocknung in einer Inertgasatmosphäre, vorzugsweise Stickstoff, oder in einer Luftatmosphäre.

2. Prozess nach Anspruch 1, **dadurch gekennzeichnet, dass** Schritt a) bei Raumtemperatur durchgeführt wird.

3. Prozess nach Anspruch 1 oder 2, **dadurch gekennzeichnet, dass** die Natriumhydroxidlösung eine Konzentration von 28,5-32,5 Gew.%, vorzugsweise 30 Gew.%, aufweist.

4. Prozess nach einem der vorstehenden Ansprüche, **dadurch gekennzeichnet, dass** der Natriumhydroxidlösung eine Wassermenge zugegeben wird, vorzugsweise eine Wassermenge, die der Ausgangsmenge an L-Tyrosin entspricht.

5. Prozess nach einem der vorstehenden Ansprüche, **dadurch gekennzeichnet, dass** Schritt b) in einer Inertgasatmosphäre, vorzugsweise Stickstoff, durchgeführt wird.

6. Prozess nach Anspruch 5, **dadurch gekennzeichnet, dass** die Einlasstemperatur zwischen 130 °C und 180 °C und die Auslasstemperatur zwischen 80 °C und 100 °C liegt.

7. Prozess nach einem der Ansprüche 1 bis 4, **dadurch gekennzeichnet, dass** Schritt b) in einer Luftatmosphäre durchgeführt wird.

8. Prozess nach Anspruch 7, **dadurch gekennzeichnet, dass** die Einlasstemperatur zwischen 165 °C und 180 °C, vorzugsweise 165 °C, und die Auslasstemperatur zwischen 80 und 100 °C, vorzugsweise 95 °C, liegt.

## Revendications

1. Procédé de production de tyrosine disodique, de préférence dihydratée, comprenant les étapes suivantes :
a) la préparation d'une solution aqueuse de L-tyrosine dans de l'hydroxyde de sodium ;
b) la soumission de la solution obtenue à un séchage par pulvérisation dans une atmosphère de gaz inerte, de préférence l'azote, ou dans une atmosphère d'air.

2. Procédé selon la revendication 1, **caractérisé en ce que** l'étape a) est réalisée à température ambiante.

3. Procédé selon la revendication 1 ou 2, **caractérisé en ce que** la solution d'hydroxyde de sodium est à une concentration comprise entre 28,5 % et 32,5 % en poids, de préférence de 30 % en poids.

4. Procédé selon l'une quelconque des revendications précédentes, **caractérisé en ce qu'**une quantité d'eau est ajoutée à la solution d'hydroxyde de sodium, de préférence une quantité d'eau égale à la quantité initiale de L-tyrosine.

5. Procédé selon l'une quelconque des revendications précédentes, **caractérisé en ce que** l'étape b) est réalisée dans une atmosphère de gaz inerte, de préférence l'azote.

6. Procédé selon la revendication 5, **caractérisé en ce que** la température d'entrée est comprise entre 130 °C et 180 °C et la température de sortie est comprise entre 80 et 100 °C.

7. Procédé selon l'une quelconque des revendications 1 à 4, **caractérisé en ce que** l'étape b) est réalisée dans une atmosphère d'air.

8. Procédé selon la revendication 7, **caractérisé en ce que** la température d'entrée est comprise entre 165 °C et 180 °C, de préférence 165 °C, et la température de sortie est comprise entre 80 et 100 °C, de préférence 95 °C.
